# EUROPEAN PATENT SPECIFICATION

(11) **EP 2 642 932 B1**
(45) Date of publication and mention of the grant of the patent: **19.03.2014**
(21) Application number: 11754804.0
(22) Date of filing: 31.08.2011
(51) Int. Cl.: A61B 17/32, A61B 17/3207, A61M 25/10, A61B 17/22

(54) **SCORING BALLOON AND METHOD OF MAKING SAME**
SCORING-BALLON UND VEFAHREN ZU SEINER HERSTELLUNG
BALLONNET INCISEUR ET SON PROCÉDÉ DE FABRICATION

(30) Priority: 22.11.2010 GB 201019765
(43) Date of publication of application: 02.10.2013
(73) Proprietor: Cook Medical Technologies LLC, Bloomington, IN 47404 (US)
(72) Inventor: AGGERHOLM, Steen, DK-4660 St. Heddinge (DK); ELGAARD, Per, DK-4690 Haslev (DK); HJORT, Kjeld, DK-4684 Holmegaard (DK); LYSGAARD, Thomas, DK-2680 Solroed Strand (DK)
(74) Representative: Jehan, Robert
(86) International application number: PCT/US2011/049968
(87) International publication number: WO 2012/071095

(56) References cited:
- WO-A1-2009/114425
- US-A- 5 616 149
- US-A1- 2005 137 615
- US-A1- 2007 060 863
- US-A1- 2008 097 302

## Description

### Technical Field

The present invention relates to a scoring or cutting balloon and to a method of making a scoring or cutting balloon.

### Background of the Invention

Scoring or cutting balloons are known for use in opening vessels which have become constricted or closed as a result, for example, of stenosis, plaque or other build up. The function of such balloons is to break up the material causing the constriction into fragments, which may then be collected for removal from within the patient's vasculature, for instance by use of a filter or aspiration device.

Cutting or scoring balloons have been proposed in a variety of forms, primarily by fixing of a plurality of cutting blades to a balloon or by forming integrally with the balloon radially extending solid protrusions.

There are various considerations with regard to the design and construction of cutting balloons, including the need to retain balloon flexibility, particularly during the introduction of the balloon into the patient's vasculature, the need to protect healthy vessel tissue and the balloon from the cutting elements, the reliability of the device, ease of manufacture and so on.

WO-2009/114425 discloses a method of making a cutting balloon from a raw tubing having a plurality of ribs extending along the raw tubing. When the raw tubing is inflated the ribs from scoring elements extending along the entire length of the balloon.

US-2007/060863 discloses a cutting balloon including a balloon body portion, proximal and distal end cone portions and a plurality of cutting elements extending across the body portion, each cutting element having a uniform height.

Some examples of cutting balloon can be found in US-5,733,301, US-5,797,935, US-6,129,706, US-7,306,616, US-2001/0,011,178, and WO-98/14233.

### Summary of the Invention

The present invention seeks to provide an improved cutting or scoring balloon and an improved method of making a cutting or scoring balloon. Hereinafter the term scoring is used to denote both scoring and cutting as used in the art.

According to an aspect of the present invention, there is provided a scoring balloon structure including a balloon provided with a balloon body portion, proximal and distal end cone portions extending from respective ends of the body portion, proximal and distal neck portions extending respectively from the proximal and distal end cone portions, and at least one scoring element extending across the body portion and the proximal and distal end cone portions, the or each scoring element having a height; wherein the height of the or each scoring element decreases along the taper of the proximal and distal end portions.

The provision of a scoring element which extends over the entirety of the body as well as along the cone portions of the balloon can ensure that there are no sharp edges to the ends of the scoring elements which may snag against and potentially damage healthy vessel walls or a medical device located in the patient's vasculature. The decreasing height of the scoring element or elements along the taper of the cone portions ensures that these reduce in stiffness along the cone portions and thus do not adversely affect the flexibility of the balloon. This structure of scoring element also provides important manufacturing advantages as described below.

It is preferred that the or each scoring element has a substantially even height along the balloon body portion.

The term height as used herein is intended to denote the distance by which the scoring element extends from the surface upon which it is located, be it the balloon body portion, cone portions or end portions.

In a preferred embodiment, the or each scoring element is integral with the balloon. Advantageously, the or each scoring element is formed as a single piece with the balloon and most preferably is formed of the same material as the balloon.

The height of the or each scoring element may decrease in a gradual manner from the wider end to the narrower end of the proximal and distal end cone portions of the balloon and preferably does so in a substantially linear manner. This ensures that no portion of the scoring elements presents a sudden change in height and thus a shoulder or discontinuity which can snag on the vessel wall of a medical device.

It is preferred that the height of the or each scoring element is reduced to substantially zero at the proximal and distal neck portions of the balloon. In this manner, the very ends of the balloon, including of the neck portions, are kept as flexible as possible. These areas of the balloon do not normally perform any significant cutting or scoring function and thus do not require any scoring element. It is envisaged that in some embodiments the or each scoring element could taper to zero height before the end of the cone portions of the balloon.

Advantageously, the or each scoring element extends substantially linearly along the balloon. In another embodiment, at least a portion of the or each scoring element may extend non-linearly along the balloon. In some applications, for instance, it may be preferable to have the or at least one scoring element arranged helically or annularly around the circumference of the balloon, for instance, particularly along the balloon body portion.

It is preferred that there are provided at least three or four scoring elements.

Advantageously, the or each scoring element is a co-extrusion with the balloon.

According to another aspect of the present invention, there is provided a method of forming a scoring balloon including the steps of providing a raw material form including a tube with one or more ribs extending along the length of the tube; locating the tube in a mold; constricting first and second ends of the tube and inflating the tube so as to produce a balloon provided with a balloon body portion, first and second balloon end cone portions extending from respective ends of the body portion, and first and second neck portions, and one or more scoring elements extending from said first end to said second end; and reducing the height of the scoring elements along the first and second end cone portions such that the height of the scoring elements reduces in the direction of taper of said end cone portions.

Preferably, the height of the or each scoring element is reduced in a gradual manner along the taper of the proximal and distal end cone portions of the balloon.

Advantageously, the or each rib which forms a scoring element is coextruded with the balloon.

### Brief Description of the Drawings

Embodiments of the present invention are described below, by way of example only, with reference to the accompanying drawings, in which:
Figure 1 is a side elevational view in schematic form of an embodiment of scoring balloon;
Figure 2 is a view of one end of another embodiment of scoring balloon;
Figure 3 is a view of one end of another embodiment of scoring balloon; and
Figures 4A and 4B are perspective and end views respectively of an embodiment of raw material tubing used to produce the balloons of Figures 1, 2 and 3.

### Description of the Preferred Embodiments

The preferred embodiments described herein are based upon the provision of scoring elements which are integral with the balloon itself, most preferably being of the same material as the balloon and formed as a unitary and singular structure with the balloon, for instance by co extrusion. As described below, this structure can optimise the manufacture of the scoring balloon as well as optimising the integrity and performance of the overall structure. Nevertheless, some embodiments may use scoring elements which are separate elements attached, for instance, by bonding or melting to the balloon itself. It is also envisaged that the elements could be integrally formed with the balloon but be made of a different material from that of the balloon.

The primary embodiments described below and shown in the drawings have three scoring elements extending longitudinally along the length of the balloon and being substantially equally spaced circumferentially around the balloon. This arrangement, although preferred, is not to be considered as limiting as other embodiments are also envisaged and described below.

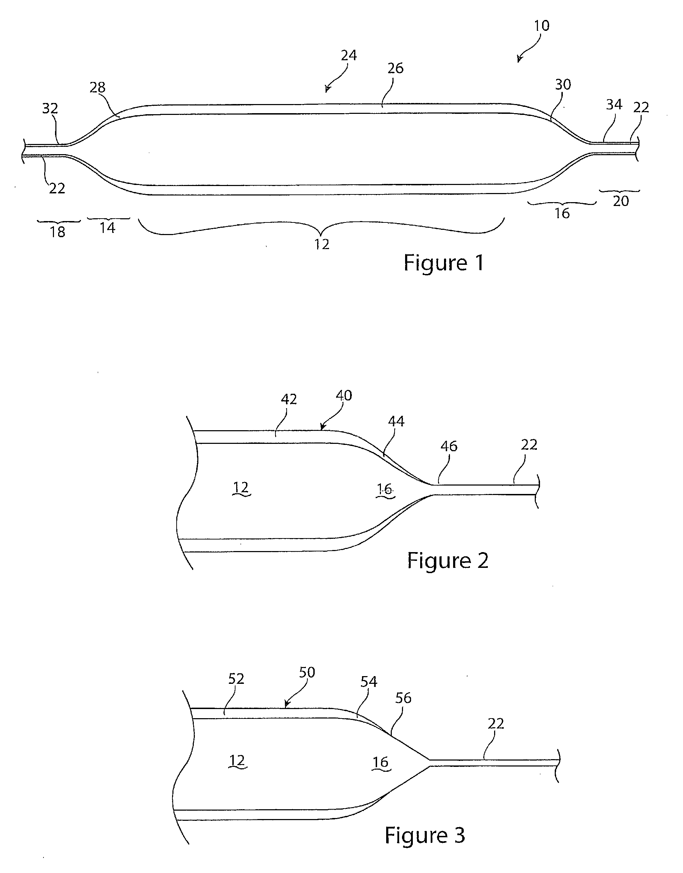

Referring to Figure 1, there is shown in schematic form a first embodiment of scoring balloon 10, which can be formed of a conventional material, compliant or relatively non-compliant. Examples of balloon material include nylon, preferably nylon 12, Pebax, polyurethane, PET, PE, and similar materials, and furthermore also including co-extrusions and blends of more than one of these materials.

The balloon 10 includes, in this embodiment, a generally cylindrical and straight body portion 12 which extends along a longitudinal axis of the balloon 10. At both ends of the body portion 12 there are provided end cone portions, 14 and 16 respectively. Although shown in the drawings as conical, the skilled person will know that in practice the end cone portions 14, 16 will typically be slightly rounded since they form part of the inflatable structure of the balloon.

At both extremities of the end cone portions 14, 16 there are neck portions 18, 20, which are fixed to a catheter or carrier element 22, typically in fluid tight manner.

As described below in further detail, the portions 12-20 of the balloon 10 are typically formed from a common raw tubing which is inflated under heating so as to stretch into the shape shown in Figure 1. Thus, in the preferred embodiment, these portions 12-20 of the balloon 10 can be formed from the same material, although may have different wall thicknesses as a result of the different extents to which the raw tubing is inflated, that is stretched during formation of the balloon. Typically, the body portion will have a thinner balloon wall, whereas the end cone portions 14, 16 will have a wall thickness which increases in the direction of reduction of their taper. The neck portions 18, 20 will have the thickest walls. It is to be appreciated that these differences in thickness may be minimal and thus of no or little material effect to the operating characteristics of the balloon 10. In some circumstances, additional manufacturing steps may be performed to even out these walls thicknesses.

The balloon structure 10 is also provided with a plurality of scoring elements 24 which extend from one end of the balloon 10 to the other. In the preferred embodiments there may be provided three or four scoring elements, although there could be just one or more than four in other embodiments.

In this example, the scoring elements 24 extend substantially linearly along the axis of the balloon and are substantially equally spaced around the circumference of the balloon.

Each scoring element 24 has a central uniform section 26 which extends along the body portion 12 of the balloon. In the preferred embodiment, the central section 26 of each scoring element 24 has a substantially constant height for the length of the balloon body portion 12, although in other embodiments this height could vary. Each scoring element 24 is also provided with tapering sections 28, 30 which extend from the central section 26 and along the balloon end cones 14, 16. Beyond these tapering sections 28, 30, each scoring element 24 may have extremities 32, 34 extending over the balloon necks. It is to be appreciated that in some embodiments these extremities 32, 34, in the finished balloon 10, may not be visible or may have been removed, for instance by being pressed or molded into the balloon neck portions 18, 20 or by being cut away, as described below.

The tapering sections 28, 30 of each scoring element taper from a height generally consistent with the central section 26 to a lower height, in the preferred embodiment to zero height, along the direction of taper of the balloon end cones 18, 20. This reduction in height of the scoring elements 24 as the balloon narrows enhances the flexibility of the balloon at the cone portions 18, 20 and also ensures that there is no abrupt change in the height of the scoring elements 24 which could present a shoulder or other edge able to snag on a vessel wall or medical device located in a patient's lumen.

Preferably, the tapering sections 28, 30 of each scoring element 24 exhibit a gradual, advantageously linear, reduction in height; although it is not excluded that this reduction could be by small steps insufficiently large to provide any shoulder large enough to cause potential disadvantages in terms of snagging and the like.

Referring now to Figure 2, there is shown a particular embodiment of scoring elements 40. In this embodiment, the scoring elements 40 extend from a central uniform section 42 along the balloon body portion 12 to tapering sections 44 which gradually taper all the way down the balloon end cones 14, 16 to the neck portions 18, 20. The sections 46 of the scoring elements 40 which lie along the balloon neck portions18, 20 have virtually no height.

Figure 3 shows another embodiment of scoring element 50, in which the tapering sections 54, which extend from the central section 52, reduce in height to such an extent that they end, that is have no height, at a position 56 located before the narrow extremity of the cone portions 14, 16 of the balloon.

In other embodiments, the tapering sections 30, 44, 54 could have tapers which are other than linear.

It will be apparent to the skilled person that the scoring elements 24, 40, 50 may be slightly rounded at the junction between the balloon body portion 12 and the end cones 14, 16, caused by rounding of the balloon on inflation.

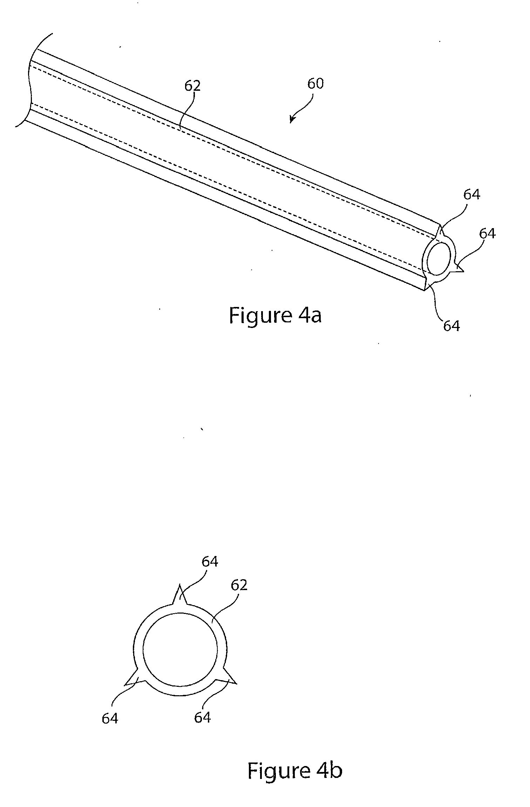

Figures 4a and 4b show an embodiment of raw tubing 60 used for the manufacture of the scoring balloons taught herein. The raw tubing 60 is advantageously a continuous length of tubing having a substantially circular cylindrical tube portion 62 from which extend radially outwardly a plurality of ribs 64. In Figures 4a and 4b the raw tubing 60 has three ribs 64 extending linearly along the outside of the tube 62, although the number will be dependent upon the number of scoring elements desired. The arrangement of the ribs 64 will also be dependent upon the desired shape of the scoring elements of the balloon. For instance, for scoring elements which extend at least partially helically around the balloon, the ribs 64 would also be at least partially helical.

In the preferred embodiment, the ribs 64 are formed to have a generally triangular or otherwise of converging shape in axial cross-section, as apparent from Figures 4a and 4b, and are preferably made of the same material as the balloon 62. Advantageously, the ribs 64 and tube 62 are extruded together, such that they are a single piece integral component with no distinction in terms of material. It is not excluded, however, that the ribs 64 could be made from a different material than that of the tube 62, for instance a material which is less flexible.

The raw tubing 60 is, on manufacture, inflated in a suitable mold under heat so as to stretch the central part of the raw tubing 60 to form the body portion 12 and end cones 14, 16 of the balloon, while end portions of the raw tubing are held radially compressed so as not to inflate and to form the neck portions 18, 20.

In an embodiment, the ribs are kept substantially intact during inflation of the raw tubing. At the of the inflation stage, the portions of the ribs which extend along the end cone and neck portions of the just formed balloon are then reduced in height, preferably by laser cutting or otherwise by ablation, grinding or other suitable method. In another embodiment, the portions of the ribs 64 which overlie the zones of the raw tubing which form the end cones 28, 30 and neck portions 32, 34 are squashed during the balloon inflation process. Specifically, the conical portions of the mold which form the end cones of the have no or reduced grooves to accommodate the ribs 64 of the raw tubing 60, as a result of which during the heating and inflation stages the ribs 64 are softened and heat squashed into the wall of the balloon.

The reduction in height of the scoring elements reduces the stiffness of these, in the preferred embodiments sufficient to offset any increase in stiffness at the end cones 14, 16 and necks 18, 20 of the balloon, caused by their greater wall thickness as a result of having been stretched less during inflation of the raw tubing 60. Thus, the preferred embodiments retain parts of the scoring elements along at least a part of the balloon end cones 14, 16, thus to maintain a scoring function even along the end cones and to avoid any unwanted increase in stiffness. Furthermore, the tapering nature of the scoring elements avoids any abrupt shoulders or edges at the ends of the scoring elements. Although in some embodiments the height of the scoring elements is reduced proportionally to the increase in wall thickness of the end cones, other embodiments may provide a different rate or nature of height reduction, some examples being disclosed above.

Laser cutting is presently a preferred method as it minimizes damage to the balloon or scoring element material and is accurate.

Reducing the height of the scoring elements after or during inflation of the raw tubing facilitates manufacture of the balloon in that it is not necessary to manufacture raw tubing with particular features along its length, which features would form the different parts of the balloon. Instead, a raw tubing which is consistent along its length enables a more efficient manufacture, for instance by extrusion of long lengths of raw tubing which can then be cut to the desired lengths either before or after inflation and formation of a balloon.

The central portions 24, 42, 52 of the scoring elements need not be of uniform height. They could, in dependence upon the characteristics desired of the balloon, have non-uniform heights, for instance to bulge outwardly towards their middle, to reduce in height towards their middle and even, in some embodiments, to have a plurality of notches along their length.

Although there are described a number of embodiments above, these are not to be understood as limiting the scope of the invention taught herein. For instance, even though the primary embodiments described above have three or four scoring elements extending longitudinally along the length of the balloon and substantially equally spaced circumferentially around the balloon, other embodiments are possible. For instance, a different number of scoring elements could be provided. Similarly, the scoring elements could be arranged non-linearly with respect to the axis of the balloon, such as helically along at least the body portion of the balloon. In other embodiments, the scoring elements could be unevenly spaced around the circumferential extent of the balloon. Various other arrangements are possible.

## Claims

1. A scoring balloon structure including a balloon (10) provided with a balloon body portion (12), proximal and distal end cone portions (14, 16) extending from respective ends of the body portion, proximal and distal neck portions (18, 20) extending respectively from the proximal and distal end cone portions (14, 16), and at least one scoring element (24) extending across the body portion and the proximal and distal end cone portions, the or each scoring element having a height; wherein the height (28, 30) of the or each scoring element decreases along the taper of the proximal and distal end portions.

2. A scoring balloon according to claim 1, wherein the or each scoring element (24) is integral with the balloon (10).

3. A scoring balloon according to claim 1 or 2, wherein the height of the or each scoring element (24) decreases in a gradual manner from the wider end to the narrower end of the proximal and distal end cone portions (14, 16) of the balloon.

4. A scoring balloon according to claim 1, 2 or 3, wherein the height of the or each scoring element (24) decreases in a substantially linear manner along the proximal and distal end cone portions (14, 16) of the balloon.

5. A scoring balloon according to any preceding claims, wherein the height of the or each scoring element (24) is reduced to substantially zero at the proximal and distal neck portions (18, 20) of the balloon.

6. A scoring balloon according to any preceding claims, wherein the or each scoring element (24) has a substantially uniform height (26) along the body portion (12) of the balloon.

7. A scoring balloon according to any preceding claim, wherein the or each scoring element (24) extends substantially linearly along the balloon (10).

8. A scoring balloon according to any one of claims 1 to 6, wherein at least a portion of the or each scoring element (24) extends non-linearly along the balloon.

9. A scoring balloon according to any preceding claim, wherein there are provided at least three scoring elements (24).

10. A scoring balloon according to any preceding claim, wherein the or each scoring element (24) is formed of the same material as the balloon (10).

11. A scoring balloon according to any preceding claim, wherein the or each scoring element (24) is a co-extrusion with the balloon (10).

12. A method of forming a scoring balloon including the steps of providing a raw material form (60) including a tube (62) with one or more ribs (64) extending along the length of the tube; locating the tube in a mold; constricting first and second ends of the tube (62) and inflating the tube so as to produce a balloon (10) provided with a balloon body portion (12), first and second balloon end cone portions (14, 16) extending from respective ends of the body portion (12), and first and second neck portions (18, 20), and one or more scoring elements (24) extending from said first end to said second end; and reducing the height of the scoring elements (24) along the first and second end cone portions (14, 16) such that the height of the scoring elements (24) reduces in the direction of taper of said end cone portions.

13. A method according to claim 12, wherein the height of the or each scoring element (24) is reduced in a gradual manner along the taper of the proximal and distal end cone portions (14, 16) of the balloon (10).

14. A method according to claim 12 or 13, wherein the or each rib (64) which forms a scoring element (24) is coextruded with the balloon.

15. A method according to any one of claims 12 to 14, wherein the height of the or each scoring element (24) is reduced by laser ablation, grinding, etching or heat flattening.

## Patentansprüche

1. Scoring-Ballonstruktur mit einem Ballon (10), der mit einem Ballonkörperabschnitt (12), proximalen und distalen Endkegelabschnitten (14, 16), die sich von den entsprechenden Enden des Körperabschnitts erstrecken, proximalen und distalen Halsabschnitten (18, 20), die sich entsprechend von den proximalen und distalen Endkegelabschnitten (14, 16) erstrecken, und mindestens einem Scoring-Element (24) bereitgestellt ist, das sich über den Körperabschnitt und die proximalen und distalen Endkegelabschnitte erstreckt, wobei das oder jedes Scoring-Element eine Höhe aufweist; wobei die Höhe (28, 30) des oder jedes Scoring-Elements entlang der Verjüngung der proximalen und distalen Endabschnitte abnimmt.

2. Scoring-Ballon nach Anspruch 1, wobei das oder jedes Scoring-Element (24) einstückig mit dem Ballon (10) ausgebildet ist.

3. Scoring-Ballon nach Anspruch 1 oder 2, wobei die Höhe des oder jedes Scoring-Elements (24) schrittweise von dem breiteren Ende zu dem schmaleren Ende der proximalen und distalen Endkegelabschnitte (14, 16) des Ballons abnimmt.

4. Scoring-Ballon nach Anspruch 1, 2 oder 3, wobei die Höhe des oder jedes Scoring-Elements (24) im Wesentlichen linear entlang der proximalen und distalen Endkegelabschnitte (14, 16) des Ballons abnimmt.

5. Scoring-Ballon nach einem der vorherigen Ansprüche, wobei die Höhe des oder jedes Scoring-Elements (24) an den proximalen und distalen Halsabschnitten (18, 20) des Ballons auf im Wesentlichen null reduziert wird.

6. Scoring-Ballon nach einem der vorherigen Ansprüche, wobei das oder jedes Scoring-Element (24) eine im Wesentlichen einheitliche Höhe (26) entlang des Körperabschnitts (12) des Ballons aufweist.

7. Scoring-Ballon nach einem der vorherigen Ansprüche, wobei das oder jedes Scoring-Element (24) sich im Wesentlichen linear entlang des Ballons (10) erstreckt.

8. Scoring-Ballon nach einem der Ansprüche 1 bis 6, wobei sich mindestens ein Abschnitt des oder jedes Scoring-Elements (24) nicht linear entlang des Ballons erstreckt.

9. Scoring-Ballon nach einem der vorherigen Ansprüche, wobei mindestens drei Scoring-Elemente (24) bereitgestellt werden.

10. Scoring-Ballon nach einem der vorherigen Ansprüche, wobei das oder jedes Scoring-Element (24) aus dem gleichen Material wie der Ballon (10) ausgebildet ist.

11. Scoring-Ballon nach einem der vorherigen Ansprüche, wobei das oder jedes Scoring-Element (24) mit dem Ballon (10) coextrudiert ist.

12. Verfahren zum Bilden eines Scoring-Ballons, das die Schritte des Bereitstellens einer Rohmaterialform (60) mit einem Rohr (62) mit einer oder mehreren Rippen (64), die sich entlang der Länge des Rohrs erstreckt/erstrecken; Anordnens des Rohrs in einer Form; Verengens der ersten und zweiten Enden des Rohrs (62) und Aufblasens des Rohrs, um einen Ballon (10) zu erzeugen, der mit einem Ballonkörperabschnitt (12), ersten und zweiten Ballonendkegelabschnitten (14, 16), die sich von den entsprechenden Enden des Körperabschnitts (12) erstrecken, bereitgestellt ist und mit ersten und zweiten Halsabschnitten (18, 20) und einem oder mehreren Scoring-Elementen (24), die sich in dem ersten Ende zu dem zweiten Ende erstrecken; und des Reduzierens der Höhe der Scoring-Elemente (24) entlang der ersten und zweiten Endkegelabschnitte (14, 16), sodass sich die Höhe der Scoring-Elemente (24) in Verjüngungsrichtung der Endkegelabschnitte reduziert, beinhaltet.

13. Verfahren nach Anspruch 12, wobei die Höhe des oder jedes Scoring-Elements (24) schrittweise entlang der Verjüngung der proximalen und distalen Endkegelabschnitte (14, 16) des Ballons (10) abnimmt.

14. Verfahren nach Anspruch 12 oder 13, wobei die oder jede Rippe (64), die ein Scoring-Element (24) bildet, mit dem Ballon coextrudiert wird.

15. Verfahren nach einem der Ansprüche 12 bis 14, wobei die Höhe des oder jedes Scoring-Elements (24) durch Laserablation, Schleifen, Ätzen oder Wärmeabflachung reduziert wird.

## Revendications

1. Structure de ballonnet inciseur comportant un ballonnet (10) muni d'une portion de corps de ballonnet (12), de portions de cône d'extrémité proximale et distale (14, 16) s'étendant depuis des extrémités respectives de la portion de corps, de portions de col proximale et distale (18, 20) s'étendant respectivement depuis les portions de cône d'extrémité proximale et distale (14, 16) et d'au moins un élément inciseur (24) s'étendant en travers de la portion de corps et des portions de cône d'extrémité proximale et distale, le ou chaque élément inciseur ayant une hauteur ; la hauteur (28, 30) du ou de chaque élément inciseur diminuant le long de la conicité des portions de cône d'extrémité proximale et distale.

2. Ballonnet inciseur selon la revendication 1, dans lequel le ou chaque élément inciseur (24) est réalisé intégralement avec le ballonnet (10).

3. Ballonnet inciseur selon la revendication 1 ou 2, dans lequel la hauteur du ou de chaque élément inciseur (24) diminue de manière progressive depuis l'extrémité plus large jusqu'à l'extrémité plus étroite des portions de cône d'extrémité proximale et distale (14, 16) du ballonnet.

4. Ballonnet inciseur selon la revendication 1, 2 ou 3, dans lequel la hauteur du ou de chaque élément inciseur (24) diminue de manière sensiblement linéaire le long des portions de cône d'extrémité proximale et distale (14, 16) du ballonnet.

5. Ballonnet inciseur selon l'une quelconque des revendications précédentes, dans lequel la hauteur du ou de chaque élément inciseur (24) diminue sensiblement jusqu'à zéro au niveau des portions de col proximale et distale (18, 20) du ballonnet.

6. Ballonnet inciseur selon l'une quelconque des revendications précédentes, dans lequel le ou chaque élément inciseur (24) présente une hauteur sensiblement uniforme (26) le long de la portion de corps (12) du ballonnet.

7. Ballonnet inciseur selon l'une quelconque des revendications précédentes, dans lequel le ou chaque élément inciseur (24) s'étend de manière sensiblement linéaire le long du ballonnet (10).

8. Ballonnet inciseur selon l'une quelconque des revendications 1 à 6, dans lequel au moins une portion du ou de chaque élément inciseur (24) s'étend de manière non linéaire le long du ballonnet.

9. Ballonnet inciseur selon l'une quelconque des revendications précédentes, dans lequel au moins trois éléments inciseurs (24) sont prévus.

10. Ballonnet inciseur selon l'une quelconque des revendications précédentes, dans lequel le ou chaque élément inciseur (24) est formé en le même matériau que le ballonnet (10).

11. Ballonnet inciseur selon l'une quelconque des revendications précédentes, dans lequel le ou chaque élément inciseur (24) est coextrudé avec le ballonnet (10).

12. Procédé de formation d'un ballonnet inciseur comportant les étapes consistant à fournir une forme en matière première (60) comportant un tube (62) avec une ou plusieurs nervures (64) s'étendant le long de la longueur du tube ; positionner le tube dans un moule ; resserrer des première et deuxième extrémités du tube (62) et gonfler le tube de manière à produire un ballonnet (10) pourvu d'une portion de corps de ballonnet (12), de première et deuxième portions de cône d'extrémité de ballonnet (14, 16) s'étendant depuis des extrémités respectives de la portion de corps (12), et de première et deuxième portions de col (18, 20), et d'un ou de plusieurs éléments inciseurs (24) s'étendant depuis ladite première extrémité jusqu'à ladite deuxième extrémité ; et réduire la hauteur des éléments inciseurs (24) le long des première et deuxième portions de cône d'extrémité (14, 16) de telle sorte que la hauteur des éléments inciseurs (24) diminue dans la direction de conicité desdites portions de cône d'extrémité.

13. Procédé selon la revendication 12, dans lequel la hauteur du ou de chaque élément inciseur (24) est réduite de manière progressive le long de la conicité des portions de cône d'extrémité proximale et distale (14, 16) du ballonnet (10).

14. Procédé selon la revendication 12 ou 13, dans lequel la ou chaque nervure (64) qui forme un élément inciseur (24) est coextrudée avec le ballonnet.

15. Procédé selon l'une quelconque des revendications 12 à 14, dans lequel la hauteur du ou de chaque élément inciseur (24) est réduite par ablation laser, par meulage, gravage ou aplatissement thermique.
